# EUROPÄISCHE PATENTSCHRIFT

(11) **EP 1 063 226 B1**
(45) Veröffentlichungstag und Bekanntmachung des Hinweises auf die Patenterteilung: **29.10.2003**
(21) Anmeldenummer: 00113328.9
(22) Anmeldetag: 23.06.2000
(51) Int. Cl.: C07C 231/08, C07C 233/47

(54) **Verfahren zur Herstellung von N-Acylaminosäuren**
Process for the preparation of N-acyl-amino acids
Procédé de préparation de N-acyle-amino-acides

(30) Priorität: 25.06.1999 DE 19929079; 14.03.2000 DE 10012251
(43) Veröffentlichungstag der Anmeldung: 27.12.2000
(73) Patentinhaber: Degussa AG, 40474 Düsseldorf (DE)
(72) Erfinder: Drauz, Karlheinz, Prof., 63579 Freigericht (DE); Burkhardt, Olaf, Dr., 63755 Alzenau-Hörstein (DE); Beller, Matthias, Prof., 18119 Rostock (DE); Eckert, Markus, Dr., 50937 Köln (DE); Moradi, Wahed, Dr., 18057 Rostock (DE); Neumann, Helfried, Dr., 18055 Rostock (DE)

(56) Entgegenhaltungen:
- DE-A- 2 115 985
- DE-C- 19 629 717
- US-A- 4 620 949

## Beschreibung

Die Erfindung betrifft ein Verfahren zur Herstellung von N-Acylaminosäuren der allgemeinen Formel I 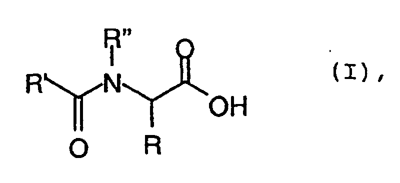 worin
R Wasserstoff, eine Carboxylgruppe, eine (C₁-C₁₂)-Alkylgruppe, die gesättigt, geradkettig, verzweigt oder zyklisch vorliegen kann, einen (C₂-C₁₂)-Alkenylrest, der ein- oder mehrfach ungesättigt, geradkettig, verzweigt oder zyklisch vorliegen kann, und eine (C₁-C₈)-Acyloxygruppe sowie einen (C₄-C₁₈)-Arylrest sowie einen (C₁-C₁₂)-Alykl-(C₄-C₁₈)-Arylrest,
R', R'' unabhängig voneinander Wasserstoff, einen gesättigten, geradkettigen, verzweigten oder zyklischen (C₁-C₂₆)-Alkyl, einen ein- oder mehrfach ungesättigten, geradkettigen, verzweigten oder zyklischen (C₂-C₂₄)-Alkenylrest, einen (C₁-C₁₂)-Alykl-(C₄-C₁₈)-Arylrest oder einen ggf. mehrfach ungesättigten (C₂-C₁₀)-Alkenyl-(C₄-C₁₈)-Arylrest, bedeuten.

N-Acylaminosäuren sind wichtige Ausgangsprodukte bei der Peptidsynthese sowie Zwischenprodukte zur Herstellung von bioaktiven Wirkstoffen. Darüber hinaus sind sie als Detergenzien, Bohrmittel-Zusatzstoffe und als Nahrungsmittelzusätze bedeutsam.

Es ist bekannt N-Acylaminosäuren durch Acylierung entsprechender Aminosäuren unter Anfall von Salznebenprodukten herzustellen. Im Falle nicht-natürlicher Aminosäuren muß die entsprechende Aminosäure vorab häufig in mehreren Stufen hergestellt werden. Ein Ein-Stufen-Verfahren, das die genannten Nachteile vermeidet, ist die Amidocarbonylierung von Aldehyden und Amiden, die im folgenden Schema dargestellt ist.

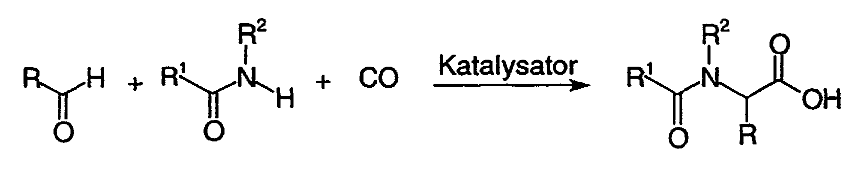

Die Amidocarbonylierung wurde zuerst von Wakamatsu et al., (Chemical Communications 1971, Seite 1540 und in DE-A2-21 15 985) beschrieben. Die Carbonylierung wird in Gegenwart von Wasserstoffgas mit einem Mol-Verhältnis CO : H₂ = 3 : 1 durchgeführt. Als Katalysator dient der Cobaltcarbonyl-Komplex Co₂(CO)₈, der in einer Konzentration von 30 mmol Co-Metall je Liter Reaktionsgemisch eingesetzt wurde.

Ein weiteres cobalt-katalysiertes Verfahren, basierend auf der Amidocarbonylierung, wird in GB 2 252 770 beschrieben. Dort gelingt die Synthese von N-Acylaminosäuren durch Umsetzung eines Carbonsäureamids mit einem Aldehyd und CO in Gegenwart eines Metallkatalysators und einer Säure als Cokatalysator.

EP-B-0 338 330 beschreibt ein Verfahren zur Herstellung von N-Acylglycinderivaten durch Verwendung eines Katalysatorsystems, bestehend aus einer Palladiumverbindung und einem ionischen Halogenid. In DE 195 45 641 und der DE 196 29 717 wird ein Verfahren zur Darstellung von N-Acylglycinderivaten aus einem Carbonsäureamid und einem Aldehyd unter Palladiumkatalyse beschrieben. Als Cokatalysatoren werden hier ionische Halogenide und zusätzlich Säure eingesetzt.

In der DE 199 20 107.2 ist die Amidocarbonylierung ausgehend von Nitrilen in Gegenwart von Palladium- bzw. Cobaltkatalysatoren beschrieben.

Aufgabe der vorliegenden Anmeldung war es, für die Amidocarbonylierung weitere Substanzen, welche besagte Reaktion katalysieren können, zur Verfügung zu stellen.

Gegenstand der Erfindung ist ein Verfahren zur Herstellung von N-Acylaminosäuren der allgemeinen Formel I 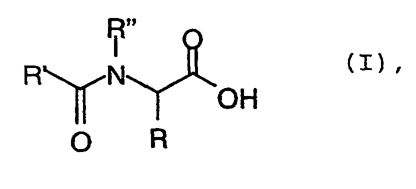 worin
R Wasserstoff, eine Carboxylgruppe, eine (C₁-C₁₂)-Alkylgruppe, die gesättigt, geradkettig, verzweigt oder zyklisch vorliegen kann, einen (C₂-C₁₂)-Alkenylrest, der ein- oder mehrfach ungesättigt, geradkettig, verzweigt oder zyklisch vorliegen kann, und eine (C₁-C₈)-Acyloxygruppe sowie einen (C₄-C₁₈)-Arylrest sowie einen (C₁-C₁₂)-Alykl-(C₄-C₁₈)-Arylrest,
R', R'' unabhängig voneinander Wasserstoff, einen gesättigten, geradkettigen, verzweigten oder zyklischen (C₁-C₂₆)-Alkyl, einen ein- oder mehrfach ungesättigten, geradkettigen, verzweigten oder zyklischen (C₂-C₂₄)-Alkenylrest, einen (C₁-C₁₂)-Alykl-(C₄-C₁₈)-Arylrest oder einen ggf. mehrfach ungesättigten (C₂-C₁₀)-Alkenyl-(C₄-C₁₈)-Arylrest, bedeuten,
welches dadurch gekennzeichnet ist, daß man ein Amid der allgemeinen Formel II

R'―CO―NH―R" (II),

R-CHO (III),

in dem R die oben angegebene Bedeutung besitzt,
in Gegenwart von Kohlenmonoxid und einem Metallkatalysator, ausgewählt aus der Gruppe der Rhodium-, Iridium- oder Ruthenium-Katalysatoren, umsetzt, wobei man bei der Reaktion ein Halogenidsalz zusetzt. Man gelangt so in vorteilhafter Weise zu den gewünschten Verbindungen der allgemeinen Formel I.

Erfindungsgemäß können als Edukte beliebige Amide als Ausgangsmaterialien verwendet werden. Beispiele für geeignete Amide sind Acetamid, Benzamid, Propionamid, N-Methylacetamid, Fettsäureamide, Acrylamid, Zimtsäureamid, Phenylessigsäureamid, Acetanilid und Harnstoff. Die Amidkomponente kann im erfindungsgemäßen Verfahren ggf. auch in situ aus entsprechenden Nitrilen beispielsweise durch säurekatalysierte Hydrolyse hergestellt werden. Beispiele für geeignete Nitrile sind Acetonitril, Benzonitril, substituierte Benzonitrile, Benzylcyanid, Acrylnitril, Malondinitril, Adiponitril, Butylcyanid, Allylcyanid, Mandelsäurenitril, und Fettsäurenitrile.

Für das erfindungsgemäße Verfahren können beliebige Aldehyde eingesetzt werden, z. B. Formaldehyd, Acetaldehyd, Propionaldehyd, Butyraldehyd, Valeraldehyd, 2-Ethylhexanal, Isobutyraldehyd, Furfural, Crotonaldehyd, Acrolein, Benzaldehyd, substituierte Benzaldehyde, Phenylacetaldehyd, 2,4-Dihydroxyphenylacetaldehyd, Glyoxalsäure und α-Acetoxypropionaldehyd. Es können auch Dialdehydverbindungen eingesetzt werden. Ebenfalls geeignet sind Substanzen, die unter den genannten Reaktionsbedingungen einen Aldehyd bilden können, z. B. Aldehydoligomere, wie Paraformaldehyd, Acetale, Allylalkohole und Epoxide.

Der Aldehyd wird zweckmäßigerweise in einer Menge von 0.5 bis 5 Equivalenten, bevorzugt 0.8 bis 2 Equivalenten, bezogen auf das Amid, eingesetzt.

Die Aldehyde können in die Reaktion in Form ihrer Trimeren oder Oligomeren eingesetzt werden.

Als aktive Metallkatalysatoren kommen für die betrachtete Reaktion im Prinzip alle dem Fachmann bekannten Katalysatoren auf Basis von Rhodium, Ruthenium oder Iridium in Frage.

Als Rhodium-, Ruthenium-, Iridiumkatalysatoren bzw. -präkatalysatoren werden vorzugsweise Metallcarbonyle bzw. Metallhalogenide bzw. Metallcarboxylate eingesetzt. Typische Katalysatoren bzw. Präkatalysatoren sind Rhodium(III)acetat, Rhodium(III)chlorid, Acetylacetonatobis(ethylen)rhodium(I), Bis(1,5-Cyclooctadien)rhodium(I)trifluormethansulfonat, Chlorobis(ethylen)rhodium(I)-dimer, Chloro(1,5-cyclooctadien)rhodium(I)-dimer, Chlorodicarbonylrhodium(I) dimer, Chlorotris(triphenylphosphan)rhodium(I), Hexarhodiumhexadecacarbonyl, Dicarbonylacetylacetonatorhodium(I), Rhodium(III)acetylacetonat, Rhodium(II)acetat-dimer, Tetrarhodiumdodecacarbonyl, Acetatodicarbonylruthenium, Bis(cyclopentadienyl)ruthenium, Dichlorobis[(p-cymene)chlororuthenium(II)], Dichloro(1,5-cyclooctadienyl)ruthenium(II), Dichlorodicarbonylbis(triphenylphosphan)ruthenium(II), Dichlorotris(triphenylphosphan)ruthenium(II), Ruthenium(III)acetylacetonat, Ruthenium(III)chlorid, Rutheniumcarbonyl, Chlorocarbonylbis(triphenylphosphan)iridium(I), Chloro-1,5-cyclooctadienyliridium(I)-dimer, Chlorotricarbonyliridium(I), Iridium(III)acetylacetonat, Iridium(III)chlorid und Iridiumcarbonyl.

Beim Einsatz der genannten Metallkatalysatoren hat sich der Zusatz von Liganden bewährt. Als Liganden finden insbesondere Phosphane wie Triarylphosphane, Trialkylphosphane und Arylalkylphosphane Verwendung. Durch Einsatz von Phosphanen mit einem oder mehreren Chiralitätszentren ist es auch möglich, in der Reaktion zu enantiomerenreinen oder mit einem Enantiomer angereicherten N-Acylaminosäuren zu gelangen.

Insbesondere eignen sich alle dem Fachmann geläufigen N-bzw. P-haltigen Liganden. Bevorzugt sind Stickstoff-Liganden wie: Phenthroline, Bisimidazoline, Benzylamine, etc., wie z. B. in A. Togni, L. M. Venanzi "Stickstoffdonoren in der Organometallchemie und in der Homogenkatalyse", Angew. Chemie, 1994, 106, 517. Auch chirale Phosphine, wie Deguphos, Ferriophos, BPPM, etc., wie z. B. in H. Brunner, W. Zettelmeier "Handbook of Enantioselective Catalysis, VCH Weinheim, 1993, oder achirale Phosphine wie Triphenylphosphan, Tri-otolylphosphan, Tricyclohexylphosphan, Tri-tertbutylphosphan, Bisdiphenylphosphinoethan, Bisdiphenylphosphinopropan, Bisdiphenylphosphinobutan oder Bisdiphenylphosphinopentan sind bevorzugt.

Die oben genannten Katalysatoren können auch als trägergebundene Katalysatoren eingesetzt werden. Als Trägermaterialien eignen sich im Prinzip alle dem Fachmann geläufigen Materialien, insbesondere aber Trägermaterialien wie Kohle, Aluminiumoxid, Titanoxid, Siliciumoxid, Bariumsulfat, etc. Besonders bevorzugt ist Kohle als Träger.

Für das erfindungsmäßige Verfahren hat sich gezeigt, daß eine Menge von 0.0001 bis 5 mol% Katalysator (berechnet auf Katalysatormetall), bevorzugt von 0.001 - 4 mol% und besonders bevorzugt von 0.01 - 2 mol% bezogen auf das Amid, ausreichend ist.

Als Halogenidsalz können z. B. Phosphoniumbromide und Phosphoniumiodide, z. B. Tetrabutylphosphoniumbromid bzw. Tetrabutylphosphoniumiodid, sowie Ammonium-, Lithium-, Natrium-, Kaliumchlorid, -bromid und -iodid verwendet werden. Bevorzugte Halogenide sind die Chloride und Bromide. Vorzugsweise wird das ionische Halogenid in einer Menge von 1 bis 100 mol%, insbesondere von 2 - 40 mol% und ganz besonders von 5 - 30 mol%, bezogen auf das Amid, eingesetzt.

In einer vorteilhaften Ausgestaltung des Verfahrens hat sich gezeigt, daß der Zusatz an Säure als weiterer Co-Katalysator häufig bessere Ergebnisse liefert. Als Säure können beispielsweise eingesetzt werden: Schwefelsäure, HCl, HBr, Trifluormethansulfonsäure, Essigsäure, Phosphorsäure, Salpetersäure, etc. Die Säure wird dabei generell in katalytischen Mengen verwendet, bevorzugt in Mengen von 0.1 - 10 mol% und besonders bevorzugt 0.5 bis 5 mol% (bezogen auf das Amid).

Geht man bei der Amidocarbonylierung von Nitrilen aus, so bietet es sich an, die Verseifung zu den Amiden ebenfalls durch oben genannte Säuren zu initiieren. Es können jedoch bevorzugt Säuren genommen werden, deren pKa-Wert < 4 ist. Bevorzugt kann man Schwefelsäure oder ein Hydrogenhalogenid, wie Hydrogenchlorid oder Hydrogenbromid, zu dieser Reaktion einsetzen. Es können auch Säuregemische solch starker Säuren eingesetzt werden. Als besonders bevorzugte Variante kann eine Mischung einer starken Säure wie z. B. Schwefelsäure oder Hydrogenbromid in Gegenwart von Ameisensäure eingesetzt werden. Die Ameisensäure kann in 1-100 Äquivalenten bezogen auf das Nitril benutzt werden.

Als Lösungsmittel für die betrachtete Reaktion können prinzipiell alle dem Fachmann geläufigen organischen Verbindungen eingesetzt werden. Bevorzugt sind dipolar aprotische Verbindungen einsetzbar. Beispiele dafür sind Dioxan, Tetrahydrofuran, N-Methylpyrrolidon, Ethylenglykoldimethylether, Essigsäureethylester, Essigsäure, Acetonitril, Benzonitril, tert.Butylmethylether, Dibutylether, Sulfolan, N,N-Dimethylacetamid oder Gemische davon. Die Lösungsmittel können in reiner Form oder produktenthaltend bzw. mit Produkt gesättigt eingesetzt werden. Bevorzugt sind N-Methylpyrrolidon, Dimethylformamid und Acetontril als Lösungsmittel.

Die Reaktion kann bei Drücken von 1 bis 250 bar, vorzugsweise von 10 bis 150 bar, und bei Temperaturen von 0 - 200 °C, vorzugsweise von 50 - 150 °C, durchgeführt werden.

Das erfindungsgemäße Verfahren kann vom Nitril ausgehend als "Eintopfverfahren" oder bevorzugt in zwei Stufen durchgeführt werden. Beim 2-Stufen-Prozeß wird das Nitril zunächst zu einer Mischung aus Wasser und einer Säure, z. B. konz. Schwefelsäure, getropft. Nach Zugabe von Lösungsmittel, Aldehyd, Katalysator und ionischem Halogenid wird das Gemisch mit Kohlenmonoxid umgesetzt. Dabei werden für den Gesamtprozeß hohe Ausbeuten an N-Acylaminosäure erreicht.

Falls gewünscht, kann das Verfahren auch in einer Stufe durchgeführt werden. Dazu löst man beispielsweise den Aldehyd, die Katalysatorverbindung und das Halogenid in dem Nitril und tropft dieses Gemisch zu der Säure/Wasser-Mischung und setzt es in Gegenwart von Kohlenmonoxid zum Endprodukt um.

Des weiteren ist es möglich, durch eine chirale Modifizierung des Metallkatalysators einen sehr einfachen Zugang zu enantiomer angereicherten N-Acylaminosäuren zu erhalten.

Unter einem (C₄-C₁₈)-Arylrest versteht man z. B. einen ggf. substituierten Phenyl-, Naphthyl-, Anthryl-, Phenanthryl-, Biphenylrest oder ein fünf-, sechs- oder siebengliedrigen Heteroaromat mit ggf. Stickstoff-, Sauerstoff- oder Schwefelatomen im Ring, wobei diese Reste mit Fluor, Chlor, Brom, Iod, OH, NO₂, CN, CO₂H, CHO, SO₃R''', SO₂R''', SOR''', NHCOR''', COR''', NHCHO, COAr, CO₂Ar, CF₃, CONH₂, CHCHCO₂R''', SiR''', POAr₂, POR''' substituiert sein können.

Unter einem (C₁-C₁₂)-Alkylrest versteht man einen Alkylrest mit einem bis zwölf C-Atomen, welcher alle Bindungsisomeren, die für einen derarteigen Rest vorstellbar sind, mit umfaßt. Dieser kann auch als Carbocyclus vorliegen. Entsprechendes gilt für den (C₁-C₂₆)-Alkylrest. Unter einem (C₂-C₁₂)-Alkenylrest versteht man einen Alkenylrest mit zwei bis zwölf C-Atomen, welcher alle Bindungsisomeren, die für einen derartigen Rest vorstellbar sind, mit umfaßt. Dieser kann auch als Carbocyclus vorliegen. Entsprechendes gilt für den (C₂-C₂₄)-Alkenylrest. Unter einem (C₁-C₈)-Acyloxyrest vesteht man eine linear oder verzweigten Alkylgruppe mit einem bis acht C-Atomen samt aller bei diesem Rest vorstellbaren Bindungsisomeren, welche über eine Carbonyloxyfunktion an das Molekül gebunden ist.

Die im Rest R, R' und R'' vorkommenden Alkyl- und Alkenylgruppen können dabei mit Fluor, Chlor, Brom, Iod, OH, NO₂, CN, CO₂H, CHO, SO₃R''', SO₂R''', SOR''', NHCOR''', COR''', NHCHO, COAr, CO₂Ar, CF₃, CONH₂, CHCHCO₂R''', SiR''', POAr₂, POR''' substituiert sein.

Die Abkürzung Ar steht für einen (C₄-C₁₈)-Arylrest. R''' bedeutet einen (C₁-C₁₂)-Alkylrest der gesättigt, gradkettig, verzweigt oder zyklisch vorliegen kann, einen (C₂-C₁₂)-Alkenylrest, der ein- oder mehrfach ungesättigt, gradkettig, verzweigt oder zyklisch vorliegen kann.

Es ist in der Literatur bekannt, daß Carbonsäureamide mit Aldehyden und Kohlenmonoxid zu N-Acylaminosäuren reagieren. Als Katalysatoren für diese Reaktion wurden bis dato nur Palladium- und Cobaltkomplexe verwendet. Vor diesem Hintergrund ist es für den Fachmann überraschend, daß auch Rhodium-, Iridium- und Rutheniumkomplexe die Reaktion von Amiden mit Aldehyden und Kohlenmonoxid katalysieren. Die Reaktionen verlaufen mit sehr hohen Selektivitäten und guten Katalysatorproduktivitäten. Nichtumgesetztes Edukt kann durch dem Fachmann geläufige Aufarbeitungsverfahren (Destillation, Kristallisation) leicht zurückgewonnen werden und ist wieder einsetzbar, so daß auch gute Ausbeuten in kontinuierlichen -Verfahren erzielt werden können.

### Beispiele

### Beispiel 1:

Eine 10%ige Lösung von 25 mmol Cyclohexylcarbaldehyd und 25 mmol Acetamid in N-Methylpyrrolidon werden mit 0.25 mol% Chloro-1,5-cyclooctadienyliridium dimer, 0.5 mol% Triphenylphosphan, 0.10 g Schwefelsäure und 35 mol% LiBr in einem 300 ml Autoklaven mit 60 bar Kohlenmonoxid bei 100°C umgesetzt. Nach einer Reaktionszeit von 12 h wird das Lösemittel im Vakuum entfernt und der Rückstand mittels HPLC analysiert.
Umsatz: 30%
Selektivität: 90% N-Acetylcyclohexylglycin
Turnover number: 108

### Beispiel 2:

Eine 10%ige Lösung von 25 mmol Cyclohexylcarbaldehyd und 25 mmol Acetamid in N-Methylpyrrolidon werden mit 0.25 mol% Chloro-1,5-cyclooctadienyliridium, 0.5 mol% Triphenylphosphan, 0.10 g Schwefelsäure und 35 mol% LiBr in einem 300 ml Autoklaven mit 60 bar Kohlenmonoxid bei 100°C umgesetzt. Nach einer Reaktionszeit von 24 h wird das Lösemittel im Vakuum entfernt und der Rückstand mittels HPLC analysiert.
Umsatz: 46%
Selektivität: 89% N-Acetylcyclohexylglycin
Turnover number: 176

### Beispiel 3:

Eine 10%ige Lösung von 25 mmol Cyclohexylcarbaldehyd und 25 mmol Acetamid in N-Methylpyrrolidon werden mit 0.25 mol% Chloro-1,5-cyclooctadienyliridium, 0.5 mol% Triphenylphosphan, 0.10 g Schwefelsäure und 35 mol% LiBr in einem 300 ml Autoklaven mit 25 bar Kohlenmonoxid bei 100°C umgesetzt. Nach einer Reaktionszeit von 12 h wird das Lösemittel im Vakuum entfernt und der Rückstand mittels HPLC analysiert.
Umsatz: 14%
Selektivität: 93% N-Acetylcyclohexylglycin
Turnover number: 52

### Beispiel 4:

Eine 10%ige Lösung von 25 mmol Cyclohexylcarbaldehyd und 25 mmol Acetamid in N-Methylpyrrolidon werden mit 0.25 mol% Carbonylchlorobis(triphenylphosphan)iridium, 0.5 mol% Triphenylphosphan, 0.10 g Schwefelsäure und 35 mol% LiBr in einem 300 ml Autoklaven mit 60 bar Kohlenmonoxid bei 100°C umgesetzt. Nach einer Reaktionszeit von 12 h wird das Lösemittel im Vakuum entfernt und der Rückstand mittels HPLC analysiert.
Umsatz: 28%
Selektivität: 93% N-Acetylcyclohexylglycin
Turnover number: 104

### Beispiel 5:

Eine 10%ige Lösung von 25 mmol Cyclohexylcarbaldehyd und 25 mmol Acetamid in N-Methylpyrrolidon werden mit 0.25 mol% Carbonylchlorobis(triphenylphosphan)iridium, 0.5 mol% Triphenylphosphan, 0.10 g Trifluoressigsäure und 1 eq LiBr in einem 300 ml Autoklaven mit 60 bar Kohlenmonoxid bei 100°C umgesetzt. Nach einer Reaktionszeit von 12 h wird das Lösemittel im Vakuum entfernt und der Rückstand mittels HPLC analysiert.
Umsatz: 28%
Selektivität: 93% N-Acetylcyclohexylglycin
Turnover number: 104

### Beispiel 6:

Eine 10%ige Lösung von 25 mmol Benzaldehyd und 25 mmol Acetamid in N-Methylpyrrolidon werden mit 0.25 mol% Chloro-1,5-cyclooctadienyliridium dimer, 0.5 mol% Triphenylphosphan, 0.10 g Schwefelsäure und 35 mol% LiBr in einem 300 ml Autoklaven mit 60 bar Kohlenmonoxid bei 100°C umgesetzt. Nach einer Reaktionszeit von 12 h wird das Lösemittel im Vakuum entfernt und der Rückstand mittels HPLC analysiert.
Umsatz: 12%
Selektivität: 96% N-Acetylphenylglycin
Turnover number: 44

### Beispiel 7:

Eine 10%ige Lösung von 25 mmol Isobutyraldehyd und 25 mmol Acetamid in N-Methylpyrrolidon werden mit 0.25 mol% Chloro-1,5-cyclooctadienyliridium dimer, 0.5 mol% Triphenylphosphan, 0.10 g Schwefelsäure und 35 mol% LiBr in einem 300 ml Autoklaven mit 60 bar Kohlenmonoxid bei 100°C umgesetzt. Nach einer Reaktionszeit von 12 h wird das Lösemittel im Vakuum entfernt und der Rückstand mittels HPLC analysiert.
Umsatz: 29%
Selektivität: 97% N-Acetylvalin
Turnover number: 112

### Beispiel 8:

Eine 10%ige Lösung von 25 mmol Cyclohexylcarbaldehyd und 25 mmol Acetamid in N-Methylpyrrolidon werden mit 0.50 mol% Ruthenium(III)chlorid, 1.0 mol% Triphenylphosphan, 0.10 g Schwefelsäure und 35 mol% LiBr in einem 300 ml Autoklaven mit 60 bar Kohlenmonoxid bei 120°C umgesetzt. Nach einer Reaktionszeit von 12 h wird das Lösemittel im Vakuum entfernt und der Rückstand mittels HPLC analysiert.
Umsatz: 11%
Selektivität: 91% N-Acetylcyclohexylglycin
Turnover number: 20

### Beispiel 9:

Eine 10%ige Lösung von 25 mmol Cyclohexylcarbaldehyd und 25 mmol Acetamid in N-Methylpyrrolidon werden mit 0.25 mol% Dichloro-tris(triphenylphosphan)ruthenium, 0.5 mol% Triphenylphosphan, 0.10 g Schwefelsäure und 35 mol% LiBr in einem 300 ml Autoklaven mit 60 bar Kohlenmonoxid bei 100°C umgesetzt. Nach einer Reaktionszeit von 12 h wird das Lösemittel im Vakuum entfernt und der Rückstand mittels HPLC analysiert.
Umsatz: 12%
Selektivität: 92% N-Acetylcyclohexylglycin
Turnover number: 22

### Beispiel 10:

Eine 10%ige Lösung von 25 mmol Cyclohexylcarbaldehyd und 25 mmol Acetamid in N-Methylpyrrolidon werden mit 0.25 mol% Rhodiumtrichlorid, 0.5 mol% Triphenylphosphan, 0.10 g Schwefelsäure und 35 mol% LiBr in einem 300 ml Autoklaven mit 60 bar Kohlenmonoxid bei 120°C umgesetzt. Nach einer Reaktionszeit von 12 h wird das Lösemittel im Vakuum entfernt und der Rückstand mittels HPLC analysiert.
Umsatz: 18%
Selektivität: 83% N-Acetylcyclohexylglycin
Turnover number: 60

### Beispiel 11:

Eine 10%ige Lösung von 25 mmol Cyclohexylcarbaldehyd und 25 mmol Acetamid in N-Methylpyrrolidon werden mit 0.25 mol% Rhodium(III)acetylacetonat, 0.10 g Schwefelsäure und 35 mol% LiBr in einem 300 ml Autoklaven mit 60 bar Kohlenmonoxid bei 120°C umgesetzt. Nach einer Reaktionszeit von 12 h wird das Lösemittel im Vakuum entfernt und der Rückstand mittels HPLC analysiert.
Umsatz: 21%
Selektivität: 86% N-Acetylcyclohexylglycin
Turnover number: 72

### Beispiel 12:

Eine 10%ige Lösung von 25 mmol Cyclohexylcarbaldehyd und 25 mmol Acetamid in N-Methylpyrrolidon werden mit 0.25 mol% Rhodium(II)acetat dimer, 0.10 g Schwefelsäure und 35 mol% LiBr in einem 300 ml Autoklaven mit 60 bar Kohlenmonoxid bei 120°C umgesetzt. Nach einer Reaktionszeit von 12 h wird das Lösemittel im Vakuum entfernt und der Rückstand mittels HPLC analysiert.
Umsatz: 20%
Selektivität: 96% N-Acetylcyclohexylglycin
Turnover number: 72

### Beispiel 13:

Eine 10%ige Lösung von 25 mmol Benzaldehyd und 25 mmol Acetamid in N-Methylpyrrolidon werden mit 0.25 mol% Rhodium(III)acetylacetonat, 0.10 g Schwefelsäure und 35 mol% LiBr in einem 300 ml Autoklaven mit 60 bar Kohlenmonoxid bei 120°C umgesetzt. Nach einer Reaktionszeit von 12 h wird das Lösemittel im Vakuum entfernt und der Rückstand mittels HPLC analysiert.
Umsatz: 21%
Selektivität: 86% N-Acetylphenylglycin
Turnover number: 72

## Patentansprüche

1. Verfahren zur Herstellung von N-Acylaminosäuren der allgemeinen Formel I 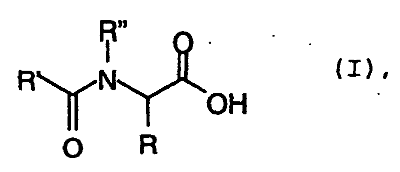 worin
R Wasserstoff, eine Carboxylgruppe, eine (C₁-C₁₂)-Alkylgruppe, die gesättigt, geradkettig, verzweigt oder zyklisch vorliegen kann, einen (C₂-C₁₂)-Alkenylrest, der ein- oder mehrfach ungesättigt, geradkettig, verzweigt oder zyklisch vorliegen kann, und eine (C₁-C₈)-Acyloxygruppe sowie einen (C₄-C₁₈)-Arylrest sowie einen (C₁-C₁₂)-Alykl-(C₄-C₁₈) -Arylrest,
R', R'' unabhängig voneinander Wasserstoff, einen gesättigten, geradkettigen, verzweigten oder zyklischen (C₁-C₂₆)-Alkyl, einen ein- oder mehrfach ungesättigten, geradkettigen, verzweigten oder zyklischen (C₂-C₂₄)-Alkenylrest, einen (C₁-C₁₂)-Alykl-(C₄-C₂₈)-Arylrest oder einen ggf. mehrfach ungesättigten (C₂-C₁₂)-Alkenyl- (C₄-C₁₈)-Arylrest, bedeuten,
**dadurch gekennzeichnet,**
**daß** man ein Amid der allgemeinen Formel II
R'―CO―NH―R" (II),
in dem R' und R'' die oben angegebene Bedeutung besitzen,
mit einem Aldehyd der allgemeinen Formel III
R―CHO (III),
in dem R die oben angegebene Bedeutung besitzt,
in Gegenwart von einer Säure, Kohlenmonoxid und einem Metallkatalysator, ausgewählt aus der Gruppe der Rhodium-, Iridium- oder Ruthenium-Katalysatoren, zu Verbindungen der allgemeinen Formel I umsetzt, wobei man bei der Reaktion ein Halogenidsalz zusetzt.

2. Verfahren nach Anspruch 1,
**dadurch gekennzeichnet,**
**daß** man den Aldehyd in einer Menge von 0.5 bis 5, bevorzugt 0.8 bis 2 Equivalenten, bezogen auf das Amid, einsetzt.

3. Verfahren nach Anspruch 1 und/oder 2,
**dadurch gekennzeichnet,**
**daß** man den Aldehyd in Form seiner Trimeren oder Oligomeren in die Reaktion einsetzt.

4. Verfahren nach Anspruch 1,
**dadurch gekennzeichnet,**
**daß** man als Metallkatalysator eine Rhodium^{0,1+,2+,3+}-Verbindung, Ruthenium^{0,2+,3+}-Verbindung oder Iridium^{0,1+,3+}-Verbindung benutzt.

5. Verfahren nach Anspruch 1 und/oder 4,
**dadurch gekennzeichnet,**
**daß** man zu dem Metallkatalysator P- bzw. N-haltige Liganden hinzufügt.

6. Verfahren nach Anspruch 4,
**dadurch gekennzeichnet,**
**daß** man die Katalysatorverbindung in einer Menge von 0,0001 bis 5 mol% bezogen auf das Amid einsetzt.

7. Verfahren nach Anspruch 6,
**dadurch gekennzeichnet,**
**daß** man die Katalysatorverbindung in einer Menge von 0,01 bis 2 mol% bezogen auf das Amid einsetzt.

8. Verfahren nach Anspruch 1,
**dadurch gekennzeichnet,**
**daß** man das Halogenidsalz in einem Konzentrationsbereich von 0,1 bis 100 mol%, bevorzugt 2 bis 40 mol%, bezogen auf das Amid einsetzt.

9. Verfahren nach einem oder mehreren der vorhergehenden Ansprüche,
**dadurch gekennzeichnet,**
**daß** man die Umsetzung in Gegenwart einer Säure mit einem pKa-Wert von < 4 durchführt.

10. Verfahren nach Anspruch 9,
**dadurch gekennzeichnet,**
**daß** man die Umsetzung in Gegenwart von Schwefelsäure oder einem Hydrogenhalogenid durchführt.

11. Verfahren nach einem der vorhergehenden Ansprüche 9 bis 10,
**dadurch gekennzeichnet,**
**daß** zusätzlich zur Säure Ameisensäure bei der Reaktion zugegen ist.

12. Verfahren nach einem oder mehreren der vorhergehenden Ansprüche,
**dadurch gekennzeichnet,**
**daß** man die Reaktion in dipolar aprotischen Lösungsmitteln oder Lösungsmittelgemischen ausführt.

13. Verfahren nach Anspruch 12,
**dadurch gekennzeichnet,**
**daß** man als Lösungsmittel N-Methylpyrrolidin, Dimethylformamid oder Acetonitril einsetzt.

14. Verfahren nach einem oder mehreren der vorhergehenden Ansprüche,
**dadurch gekennzeichnet,**
**daß** man bei einem Gasdruck von Kohlenmonoxid von 1 bis 250 bar, bevorzugt 10 - 150 bar, arbeitet.

15. Verfahren nach einem oder mehreren der vorhergehenden Ansprüche,
**dadurch gekennzeichnet,**
**daß** man bei einer Temperatur von 0 bis 200 °C, bevorzugt 50 bis 150 °C, arbeitet.

16. Verfahren nach einem oder mehreren der vorhergehenden Ansprüchen,
**dadurch gekennzeichnet,**
**daß** man das Verfahren ausgehend von Nitril in einer Stufe ausführt.

17. Verfahren nach einem oder mehreren der vorhergehenden Ansprüche,
**dadurch gekennzeichnet,**
**daß** man zur Herstellung der enantiomer angereicherten N-Acylaminosäuren chiral modifizierte Metallkatalysatoren verwendet.

## Claims

1. Process for the production of N-acylamino acids with the general formula I 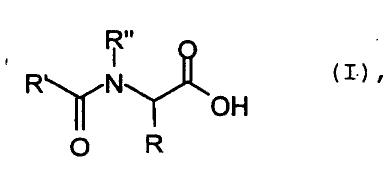 where
R denotes hydrogen, a carboxyl group, a (C₁-C₁₂) alkyl group, which may be saturated, straight-chain, branched or cyclic, a (C₂-C₁₂) alkenyl radical, which may be mono- or polyunsaturated, straight-chain, branched or cyclic, and a (C₁-C₈) acyloxy group, also a (C₄-C₁₈) aryl radical, also a (C₁-C₁₂) alkyl (C₄-C₁₈) aryl radical,
R', R'' independently denote hydrogen, a saturated, straight-chain, branched or cyclic (C₁-C₂₆) alkyl, a mono- or polyunsaturated, straight-chain, branched or cyclic (C₂-C₂₄) alkenyl radical, a (C₁-C₁₂) alkyl (C₄-C₁₈) aryl radical or an optionally polyunsaturated (C₂-C₁₀) alkenyl (C₄-C₁₈) aryl radical,
**characterised in that**
an amide with the general formula II
R'―CO―NH―R" (II),
in which R' and R'' have the meaning given above, is reacted with an aldehyde with the general formula III
R―CHO (III),
in which R has the meaning given above,
in the presence of an acid, carbon monoxide and a metal catalyst selected from the group of rhodium, iridium or ruthenium catalysts, to form compounds with the general formula I, whereby a halide salt is added during the reaction.

2. Process according to claim 1,
**characterised in that**
the aldehyde is used in a quantity of from 0.5 to 5, preferably 0.8 to 2 equivalents, relative to the amide.

3. Process according to claim 1 and/or 2,
**characterised in that**
the aldehyde is used in the reaction in the form of its trimers or oligomers.

4. Process according to claim 1,
**characterised in that**
a rhodium^{0,1+,2+,3+} compound, ruthenium^{0,2+,3+} compound or iridium^{0,1+,3+} compound is used as metal catalyst.

5. Process according to claim 1 and/or 4,
**characterised in that**
P- or N-containing ligands are added to the metal catalyst.

6. Process according to claim 4,
**characterised in that**
the catalyst compound is used in a quantity of from 0.0001 to 5 mol% relative to the amide.

7. Process according to claim 6,
**characterised in that**
the catalyst compound is used in a quantity of from 0.01 to 2 mol% relative to the amide.

8. Process according to claim 1,
**characterised in that**
the halide salt is used in a concentration range from 0.1 to 100 mol%, preferably 2 to 40 mol%, relative to the amide.

9. Process according to one of more of the above claims,
**characterised in that**
the reaction is performed in the presence of an acid with a pKa value of < 4.

10. Process according to claim 9,
**characterised in that**
the reaction is performed in the presence of sulfuric acid or a hydrogen halide.

11. Process according to one of the above claims 9 to 10,
**characterised in that**
in addition to the acid, formic acid is added during the reaction.

12. Process according to one or more of the above claims,
**characterised in that**
the reaction is performed in dipolar aprotic solvents or solvent mixtures.

13. Process according to claim 12,
**characterised in that**
N-methylpyrrolidine, dimethylformamide or acetonitrile are used as solvents.

14. Process according to one or more of the above claims,
**characterised in that**
the reaction is performed at a carbon monoxide gas pressure of from 1 to 250 bar, preferably 10 - 150 bar.

15. Process according to one or more of the above claims,
**characterised in that**
the reaction is performed at a temperature of from 0 to 200 °C, preferably 50 to 150 °C.

16. Process according to one or more of the above claims,
**characterised in that**
the process is performed in a single stage starting from nitrile.

17. Process according to one or more of the previous claims,
**characterised in that**
chirally modified metal catalysts are used to produce the enantiomer-enriched N-acylamino acids.

## Revendications

1. Procédé de préparation de N-acylaminoacides répondant à la formule générale I 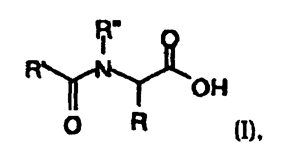 dans laquelle
R représente un atome d'hydrogène, un groupe carboxyle, un groupe alkyle en (C₁-C₁₂) qui peut être saturé, à chaîne droite, ramifié ou cyclique, un reste alcényle en (C₂-C₁₂) qui peut être une ou plusieurs fois insaturé, à chaîne droite, ramifié ou cyclique, et un groupe acyloxy en (C₁-C₈) ainsi qu'un reste aryle en (C₄-C₁₈) et un reste alkyle en (C₁-C₁₂)-aryle en (C₄-C₁₈),
R', R" représentent, indépendamment l'un de l'autre, un atome d'hydrogène, un groupe alkyle en (C₁-C₂₆) saturé, à chaîne droite, ramifié ou cyclique, un reste alcényle en (C₂-C₂₄) une ou plusieurs fois insaturé, à chaîne droite, ramifié ou cyclique, un reste alkyle en (C₁-C₁₂)-aryle en (C₄-C₁₈) ou un reste alcényle en (C₂-C₁₂)-aryle en (C₄-C₁₈), le cas échéant plusieurs fois insaturé,
**caractérisé en ce que**.
on fait réagir un amide répondant à la formule générale II
R'―CO―NH―R" (II),
dans laquelle R' et R" ont la signification ci-dessus indiquée,
avec un aldéhyde répondant à la formule générale III
R―CHO (III),
dans laquelle R possède la signification ci-dessus indiquée,
en présence d'un acide, de monoxyde de carbone et d'un catalyseur métallique choisi dans le groupe constitué des catalyseurs à base de rhodium, iridium ou ruthénium, pour obtenir des composés répondant à la formule générale I en ajoutant un halogénure au cours de la réaction.

2. Procédé selon la revendication 1,
**caractérisé en ce que**.
on met en oeuvre l'aldéhyde en une quantité de 0,5 à 5, de préférence 0,8 à 2 équivalent(s) par rapport à l'amide.

3. Procédé selon la revendication 1 et/ou 2,
**caractérisé en ce que**,
on met en oeuvre, dans la réaction, l'aldéhyde sous forme de ses trimères ou oligomères.

4. Procédé selon la revendication 1,
**caractérisé en ce que**,
on utilise comme catalyseur métallique un composé du rhodium ^{0,1+,2+,3+}, un composé du ruthénium ^{0,2+,3+} ou un composé de l'iridium ^{0,1+,3+}.

5. Procédé selon la revendication 1, et/ou 4
**caractérisé en ce que**,
on ajoute au catalyseur métallique des coordinats contenant P et N.

6. Procédé selon la revendication 4
**caractérisé en ce que**,
on utilise le composé catalyseur en une quantité de 0,0001 à 5 mole(s) % par rapport à l'amide.

7. Procédé selon la revendication 6
**caractérisé en ce que**,
on utilise le composé catalyseur en une quantité de 0,01 à 2 mole(s) % par rapport à l'amide.

8. Procédé selon la revendication 1,
**caractérisé en ce que**,
on met en oeuvre l'halogénure dans une plage de concentration de 0,1 à 100 mole(s) %, de préférence 2 à 40 moles % par rapport à l'amide.

9. Procédé selon une ou plusieurs des revendications précédentes,
**caractérisé en ce que**,
on effectue la réaction en présence d'un acide présentant un pKa < 4.

10. Procédé selon la revendication 9,
**caractérisé en ce que**,
on effectue la réaction en présence d'acide sulfurique ou d'un halogénure d'hydrogène.

11. Procédé selon l'une ou l'autre des revendications 9 et 10 précédentes,
**caractérisé en ce que**,
de l'acide formique est présent au cours de la réaction, en plus de l'acide.

12. Procédé selon une ou plusieurs des revendications précédentes,
**caractérisé en ce que**,
la réaction est effectuée dans des solvants aprotiques dipolaires ou des mélanges de solvants aprotiques dipolaires.

13. Procédé selon la revendication 12,
**caractérisé en ce que**,
on utilise comme solvant la N-méthylpyrrolidone, le diméthylformamide ou l'acétonitrile.

14. Procédé selon une ou plusieurs des revendications précédentes,
**caractérisé en ce que**,
on opère sous une pression de monoxyde de carbone de 1 à 250 bar(s), de préférence 10 - 150 bars.

15. Procédé selon une ou plusieurs des revendications précédentes,
**caractérisé en ce que**,
on opère à une température de 0 à 200 °C, de préférence 50 à 150 °C.

16. Procédé selon une ou plusieurs des revendications précédentes,
**caractérisé en ce que**,
on met en oeuvre le procédé en partant de nitrile en une seule étape.

17. Procédé selon une ou plusieurs des revendications précédentes.
**caractérisé en ce que**,
on utilise, pour la production des N-acylaminoacides enrichis en énantiomères, des catalyseurs métalliques chiraux après modification.
